# FASCICULE DE BREVET EUROPEEN

(11) **EP 0 937 043 B1**
(45) Date de publication et mention de la délivrance du brevet: **06.03.2002**
(21) Numéro de dépôt: 97948974.7
(22) Date de dépôt: 02.12.1997
(51) Int. Cl.: C07D 211/70, A61K 31/445

(54) **NOUVEAUX DERIVES TETRAHYDROPYRIDINIQUES SUBSTITUES, LEUR PROCEDE DE PREPARATION ET LES COMPOSITIONS PHARMACEUTIQUES QUI LES CONTIENNENT**
SUBSTITUIERTE TETRAHYDROPYRIDINDERIVATE, VERFAHREN ZU IHRER HERSTELLUNG UND DIESE ENTHALTENDE ARZNEIMITTEL
NOVEL SUBSTITUTED TETRAHYDROPYRIDIN DERIVATIVES, METHOD OF PREPARATION AND PHARMACEUTICAL COMPOSITIONS CONTAINING THEM

(30) Priorité: 05.12.1996 FR 9614951
(43) Date de publication de la demande: 25.08.1999
(73) Titulaire: LES LABORATOIRES SERVIER, 92200 Neuilly sur Seine (FR)
(72) Inventeur: MARAZANO, Christian, F-92260 Fontenay aux Roses (FR); COMPERE, Delphine, F-91400 Orsay (FR); DAS, C., Bhupesh, F-91190 Gif sur Yvette (FR); LEPAGNOL, Jean, F-28210 Chaudon (FR)
(86) Numéro de dépôt international: FR9702172
(87) Numéro de publication internationale: WO9824765

(56) Documents cités:
- DRIESSENS, FRANK ET AL: "Sedum alkaloids. XI. Synthesis of sedinone and sedacrine by application of anodic oxidation" CAN. J. CHEM. (1991), 69(2), 211-17 CODEN: CJCHAG;ISSN: 0008-4042, 1991, XP002037102 cité dans la demande
- H. E. J. RES: "Andrachine, an alkaloid from Andrachne aspera" PHYTOCHEMISTRY, vol. 26, no. 2, 1987, pages 585-586, XP002037103
- WILLIAMS, H. ET. AL.: ".delta.-3-Piperidine alkaloids from the toxic plant Lobelia berlandieri" J. AGRIC. FOOD CHEM., vol. 35, no. 1, 1987, pages 19-22, XP002037104
- CHEMICAL ABSTRACTS, vol. 105, no. 5, 4 août 1986 Columbus, Ohio, US; abstract no. 43121, NATSUME, MITSUTAKA: "Synthesis of alkaloids containing piperidine ring" XP002037107 & YUKI GOSEI KAGAKU KYOKAISHI (1986), 44(4), 326-39 CODEN: YGKKAE;ISSN: 0372-770X, 1986,
- BAILEY, THOMAS R. ET AL: "Total synthesis of anhydrocannabisativene" J. AM. CHEM. SOC. (1984), 106(11), 3240-5 CODEN: JACSAT;ISSN: 0002-7863, 1984, XP002037105
- COLAU, B.; HOOTELE, C.: "Sedum alkaloids" CAN. J. CHEM., vol. 61, no. 3, 1983, pages 470-472, XP002037106

## Description

La présente invention concerne de nouveaux dérivés tétrahydropyridiniques substitués, leur procédé de préparation et les compositions pharmaceutiques qui les contiennent.

De nombreux travaux de la littérature décrivent la synthèse de composés dérivés de la lobéline en tant qu'alcaloïdes susceptibles d'interagir avec les récepteurs nicotiniques. On peut en particulier citer Hootelé pour la synthèse de la sédinone et la sédacrine (Hootelé C., Driessens F., Can. J. Chem., 1991, 69 (2), pp. 211-17) et Natsume pour la synthèse de la sédinine (Natsume M., Ogawa M., Hétérocycles, 1985, 23 (4), pp. 831-4).

Les composés de la présente invention sont nouveaux et particulièrement intéressants d'un point de vue pharmacologique pour leur interaction spécifique avec les récepteurs nicotiniques, trouvant leur application dans le traitement de maladies associées au vieillissement cérébral.

Le vieillissement de la population par augmentation de l'espérance de vie a entraîné parallèlement un large accroissement de l'incidence des maladies neurodégénératives liées à l'âge et notamment de la maladie d'Alzheimer. Les principales manifestations cliniques du vieillissement cérébral et surtout des maladies neurodégénératives sont les déficits des fonctions mnésiques et cognitives qui peuvent conduire à la démence. il est largement démontré que parmi les différents neurotransmetteurs, l'acétylcholine tient une place prépondérante dans les fonctions de mémoire et que les voies neuronales cholinergiques sont dramatiquement détruites lors de certaines maladies neurodégénératives ou en déficit d'activation lors du vieillissement cérébral. C'est pourquoi de nombreuses approches thérapeutiques ont visé à empêcher la destruction du neuromédiateur via l'acétylcholine-estérase ou ont cherché à se substituer au neuromédiateur déficitaire. Dans ce dernier 0cas, les agonistes cholinergiques proposés ont été de type muscarinique, spécifiques pour les récepteurs post-synaptiques M1.

Récemment, il a été montré qu'en fait l'atteinte cholinergique liée à la maladie d'Alzheimer touchait davantage les neurones portant les récepteurs nicotiniques (N) que ceux portant les récepteurs muscariques (Schroder et al., in « Alzheimer disease: therapeutic strategies », ed. Birkhauser Boston, 1994, 181-185). De plus, de nombreuses études ont démontré que la nicotine possède des propriétés facilitatrices de la mémoire (Warburton, Prog. Neuropsychopharmacol, 1992, 16, 181-191) et que ces propriétés s'exercent tout autant sur les fonctions mnésiques (Levin et Torry, Psychopharmacol., 1996, 123, 88-97) que sur les facultés d'attention et de vigilance (Turchi et al., Psychopharmacol., 1995, 118, 195-205). Par ailleurs, la nicotine exerce des effets neuroprotecteurs vis à vis d'agents excitotoxiques tel que le glutamate (Akaike et al., Brain Res., 1994, 644, 181-187). L'ensemble de ces données est très probablement à relier avec les études épidémiologiques qui ont montré une moindre incidence de maladie d'Alzheimer ou de Parkinson chez les sujets fumeurs.

Bien que les récepteurs nicotiniques ne soient pas encore parfaitement classifiés, il est bien démontré aujourd'hui que ces récepteurs peuvent être de natures différentes et conjointement peuvent avoir des propriétés électrophysiologiques très diverses (pour revue: Williams et al., Drug News Perspect., 1994, 7, 205-223). C'est pourquoi la nicotine possède à la fois des propriétés bénéfiques promnésiques démontrées chez le patient Alzheimer (Wilson et al., 1995, 51, 509-514) et des propriétés délétères d'accoutumance par activation de récepteurs à cinétiques d'inactivation variables.

Indépendamment de la nicotine elle-même, d'autres agonistes nicotiniques ont été décrits dont la lobéline qui possède des propriétés promnésiques puissantes (Decker et al., Pharmacol. Biochem. Behav., 45, 571-576) mais contrairement à la nicotine, qui se lie préférentiellement à certains sous-types de récepteurs à désensibilisation rapide, nommés α7 (Marks et al., J. Pharmacol. Exp. Ther., 1986, 30, 427-436). C'est pourquoi la lobéline est potentiellement dépourvue de potentiel addictif.

Les composés de la présente invention ont donc été synthétisés en tant que ligands nicotiniques possédant des propriétés promnésiques pour le traitement des déficits de mémoire associés au vieillissement cérébral et aux maladies neurodégénératives telles que la maladie d'Alzheimer, la maladie de Parkinson, la maladie de Pick, la maladie de Korsakoff et les démences frontales et sous-corticales.

Plus spécifiquement, la présente invention concerne les composés de formule (I) : 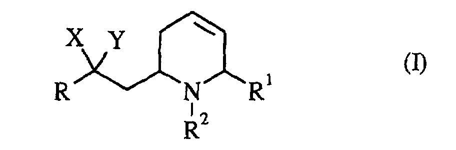 dans laquelle :
- X représente un atome d'hydrogène,
- Y représente un groupement hydroxy,
   ou X et Y forment ensemble un groupement oxo,
- R représente un groupement alkyle (C₁-C₆) linéaire ou ramifié ou un groupement phényle éventuellement substitué par un ou plusieurs atomes d'halogène ou un ou plusieurs groupements alkyle (C₁-C₆) linéaire ou ramifié, alkoxy (C₁-C₆), polyhalogéno alkyle (C₁-C₆) linéaire ou ramifié, ou hydroxy,
- R¹ représente un atome d'hydrogène ou un groupement de formule (II) : 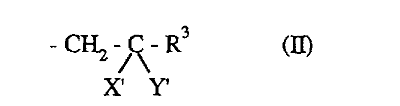 dans laquelle :
   X' représente un atome d'hydrogène,
   Y' représente un groupement hydroxy,
   ou X' et Y' représentent simultanément un groupement alkoxy (C₁-C₆) linéaire ou ramifié,
   ou X' et Y' forment ensemble un groupement oxo ou un groupement alkylène dioxy (C₁-C₄) linéaire ou ramifié,
   R³ représente un groupement alkoxy (C₁-C₆) linéaire ou ramifié, ou un groupement phényle éventuellement substitué par un ou plusieurs atomes d'halogène, ou un ou plusieurs groupements alkyle (C₁-C₆) linéaire ou ramifié, alkoxy (C₁-C₆), polyhalogénoalkyle (C₁-C₆) linéaire ou ramifié, ou hydroxy,
- R² représente un atome d'hydrogène, un groupement alkyle (C₁-C₆) linéaire ou ramifié, éventuellement substitué par un ou plusieurs groupements, identiques ou différents, aryle éventuellement substitué, alkoxy (C₁-C₆) linéaire ou ramifié, ou hydroxy, étant entendu que lorsque R et R² représentent simultanément un groupement méthyle, X représente un atome d'hydrogène, Y représente un groupement hydroxy et R¹ représente un groupement de formule (II) où X' représente un atome d'hydrogène et Y' un groupement hydroxy, alors R³ ne peut représenter un groupement phényle,
   leurs isomères ainsi que leurs sels d'addition à un acide ou à une base pharmaceutiquement acceptable.

Par aryle éventuellement substitué, on entend phényle ou naphtyle, chacun de ces groupements étant éventuellement substitué par un ou plusieurs atomes d'halogène ou un ou plusieurs groupements alkyle (C₁-C₆) linéaire ou ramifié, alkoxy (C₁-C₆), polyhalogéno alkyle (C₁-C₆) linéaire ou ramifié, ou hydroxy.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

La présente invention concerne préférentiellement les composés de formule (I) pour lesquels X représente un atome d'hydrogène et Y un groupement hydroxy, et plus particulièrement les composés de formule (I) pour lesquels X représente un atome d'hydrogène, Y un groupement hydroxy et R¹ représente un atome d'hydrogène, ou un groupement de formule (II) : 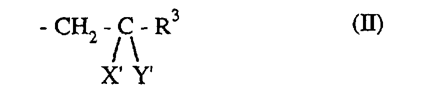 où X', Y' et R³ sont tels que définis précédemment.

L'invention s'étend également au procédé de préparation des composés de formule (I) caractérisé en ce que l'on utilise comme produit de départ un composé de formule (III) : 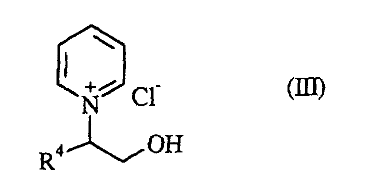 dans laquelle R⁴ représente un groupement alkyle (C₁-C₆) linéaire ou ramifié ou aryle éventuellement substitué,
que l'on fait réagir avec du borohydrure de sodium en présence d'hydroxyde de sodium pour obtenir le composé de formule (IV) : 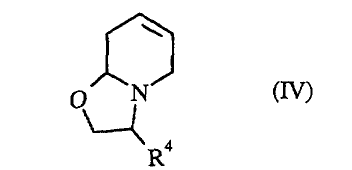 dans laquelle R⁴ est tel que défini précédemment,
qui, soumis au réactif de Réformatsky BrZnCH₂COOEt, conduit au composé de formule (V): 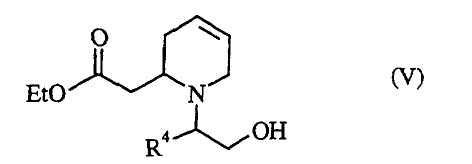 dans laquelle R⁴ a la même définition que précédemment,
qui est successivement soumis à l'action du diméthylthexylchlorosilane puis à celle de l'hydrure de lithium et d'aluminium, pour conduire au composé de formule (VI) : 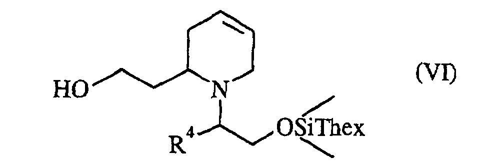 dans laquelle R⁴ est tel que défini précédemment, et Thex représente un groupement (1,1,2)-triméthyl-propyl, qui est oxydé selon Swern, pour conduire au composé de formule (VII): 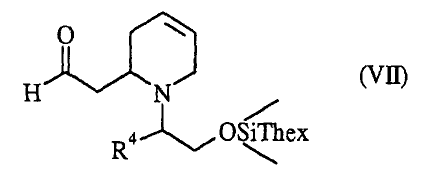 dans laquelle R⁴ et Thex sont définis comme précédemment,
qui est soumis à l'action du magnésien RMgBr où R est tel que défini dans la formule (I), pour conduire au composé de formule (VIII) : 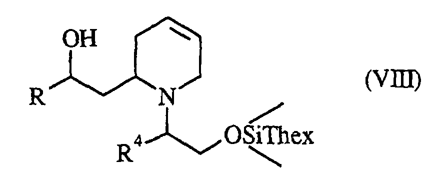 dans laquelle R, R⁴ et Thex sont tels que définis précémment,
qui est éventuellement soumis à un agent oxydant, pour conduire au composé de formule (IX) : 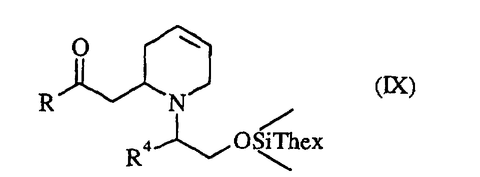 dans laquelle R, R⁴ et Thex ont la même définition que précédemment,
l'ensemble des composés (VIII) et (IX) formant le composé de formule (X) : 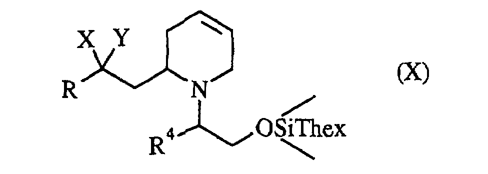 dans laquelle R, R⁴, X, Y et Thex sont tels que définis précédemment,
composé de formule (X) qui est hydrolysé en milieu acide, pour conduire au composé de formule (I/a), cas particulier des composés de formule (I) : 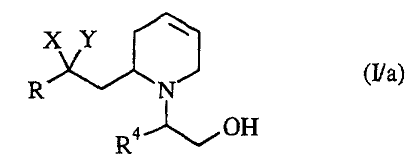 dans laquelle R, X, Y et R⁴ sont tels que définis précédemment,
qui est :
- soit traité, lorsque X représente un atome d'hydrogène et Y un groupement hydroxy, par de l'hydrogène en présence de palladium sur charbon, pour obtenir le composé de formule (I/b), cas particulier des composés de formule (I) : 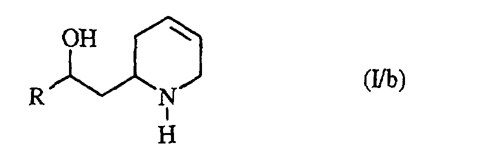 dans laquelle R est tel que défini précédemment,
- soit traité par un diphénylalkylsulfonium tétrafluoroborate de formule (XI): 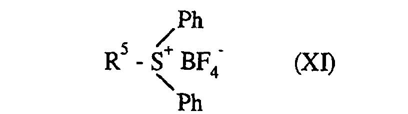
dans laquelle R⁵ représente un groupement alkyle (C₁-C₆) linéaire ou ramifié,
pour aboutir au composé de formule (XII): 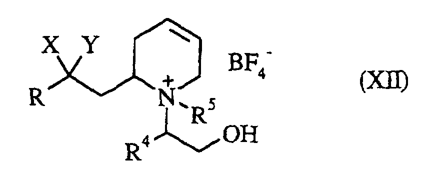 dans laquelle R, R⁴, R⁵, X et Y sont tels que définis précédemment,
composé de formule (XII) qui est soumis à l'action du tertbutanolate de potassium dans du tertbutanol, pour conduire au composé de formule (I/c), cas particulier des composés de formule (I): 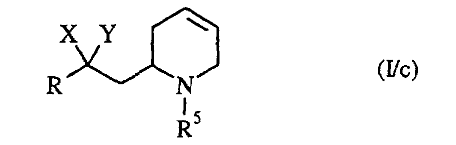 dans laquelle R, X, Y et R⁵ sont tels que définis précédemment,
qui est, dans le cas où X représente un atome d'hydrogène et Y un groupement hydroxy traité
par l'acide métachloroperbenzoïque, pour obtenir le composé de formule (XIII) : 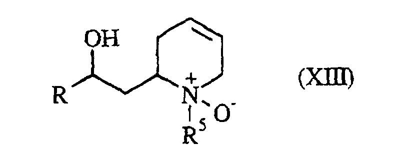 dans laquelle R et R⁵ ont la même définition que précédemment,
qui est soumis à l'action de l'anhydride trifluoroacétique, pour conduire au composé de formule (XIV) : 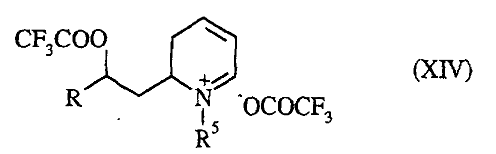 dans laquelle R et R⁵ sont tels que définis précédemment,
que l'on traite par le réactif de Réformatsky BrZnCH₂COOR⁶ où R⁶ représente un groupement alkyle (C₁-C₆) linéaire ou ramifié, pour obtenir le composé de formule (I/d), cas particulier des composés de formule (I) : 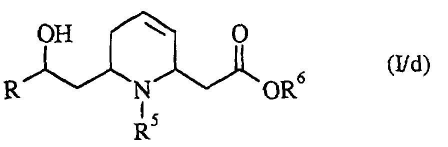 dans laquelle R, R⁵ et R⁶ ont la même définition que précédemment,
qui est successivement soumis au réactif de Weinreb 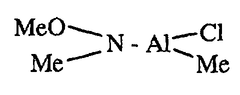 puis à l'action d'un lithien R⁷-Li où R⁷ représente un groupement phényle éventuellement substitué par un ou plusieurs atomes d'halogène ou un ou plusieurs groupements alkyle (C₁-C₆) linéaire ou ramifié, alkoxy (C₁-C₆), polyhalogénoalkyle (C₁-C₆) linéaire ou ramifié, hydroxy, pour conduire au composé de formule (I/e), cas particulier des composés de formule (I): 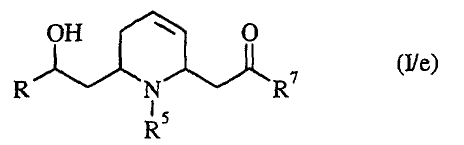 dans laquelle R, R⁵ et R⁷ sont tels que définis précédemment,
qui est :
- soit soumis à un agent oxydant, pour conduire au composé de formule (I/f), cas particulier des composés de formule (I) : 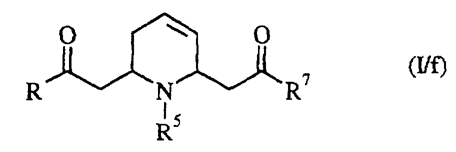 dans laquelle R, R⁵ et R⁷ ont la même définition que précédemment,
- soit soumis à un agent réducteur, pour conduire au composé de formule (I/g), cas particulier des composés de formule (I): 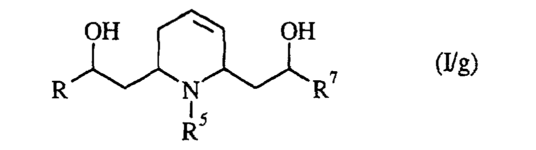 dans laquelle R, R⁵ et R⁷ sont tels que définis précédemment,
- soit protégé sous forme d'acétal par l'action d'un alcool en milieu acide, pour obtenir le composé de formule (I/h), cas particulier des composés de formule (I) : 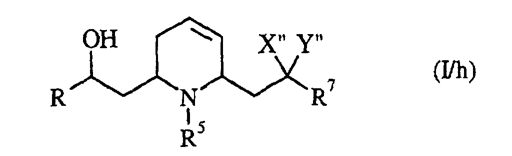
dans laquelle R, R⁵ et R⁷ sont tels que définis précédemment, et X" et Y" représentent simultanément un groupement alkoxy (C₁-C₆) linéaire ou ramifié ou forment ensemble un groupement alkylènedioxy (C₁-C₄) linéaire ou ramifié,
les composés (I/a) à (I/h) formant l'ensemble des composés de l'invention que l'on purifie, le cas échéant, selon une technique classique de purification, qui peuvent, si on le désire, être séparés en leurs différents isomères optiques ou salifiés avec un acide ou une base pharmaceutiquement acceptable.

La présente invention concerne également les intermédiaires de synthèses de formule (V), (XII) et (XIII) décrits dans le procédé précédemment explicité.

Les sels de pyridinium de départ utilisés dans le procédé précédemment décrit sont accessibles à l'homme du métier selon des procédés bien connus dans la littérature comme par exemple celui décrit par : Y. Génisson, C. Marazano, M. Mehmandoust, D. Gnecco et B.C. Das, Synlett, 1992, p. 431.

Les composés de formule (I) possèdent des propriétés pharmacologiques intéressantes.

Par leurs propriétés facilitatrices de la mémoire et grâce à leur affinité préférentielle pour les récepteurs nicotiniques α7, les composés de la présente invention sont donc utiles pour le traitement des déficits de mémoire liés au vieillissement cérébral et aux maladies neurodégénératives telles que la maladie d'Alzheimer, la maladie de Parkinson, la maladie de Pick, la maladie de Korsakoff et les démences frontales et sous-corticales.

La présente invention a également pour objet les compositions pharmaceutiques contenant les produits de formule (I), leurs isomères optiques ou un de leurs sels d'addition à une base ou un acide pharmaceutiquement acceptables, seul ou en combinaison avec un ou plusieurs excipients ou véhicules inertes, non toxiques.

Parmi les compositions pharmaceutiques selon l'invention, il sera cité plus particulièrement celles qui conviennent pour l'administration orale, parentérale, nasale, rectale, perlinguale, oculaire ou respiratoire, et notamment les comprimés simples ou dragéifiés, les comprimés sublinguaux, les sachets, les paquets, les gélules, les glossettes, les tablettes, les suppositoires, les crèmes, les pommades, les gels dermiques, les préparations injectables ou buvables, les aérosols, les gouttes oculaires ou nasales.

La posologie utile varie selon l'âge et le poids du patient, la voie d'administration, la nature de l'indication thérapeutique et des traitements éventuellement associés et s'échelonne entre 1 et 500 mg par jour en une ou plusieurs administrations.

Les exemples suivants illustrent l'invention mais ne la limitent en aucune façon.

### EXEMPLE 1 : (1S,1'S,2"S)-2-[1-(2-Hydroxy-1-phényl-éthyl)-1,2,3,6-tétrahydropyridin-2-yl]-1-phényl-éthanol

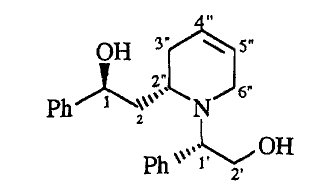

### Stade A : (3S,8aS)-3-Phényl-2,3,8,8a-tétrahydro-[5H]-oxazolo[3,2-a]pyridine

42,5 mmoles de chlorure de (1'S)-1-(2-hydroxy-1-phényl-éthyl)-pyridinium dissoutes dans l'eau sont ajoutées lentement à 132 mmoles de borohydrure de sodium en solution dans un système biphasique de soude 5N (100 ml) et d'éther (400 ml) à température ambiante.

Après une heure d'agitation magnétique vigoureuse, la phase organique est décantée et filtrée rapidement sur une courte colonne d'alumine (60 g). Après évaporation du solvant sous pression réduite, on obtient l'oxazolidine.
*Huile rouge orangé*

### Stade B : (1'S,2"S)-[1-(2-Hydroxy-1-phényl-éthyl)-2-(éthoxycarbonyl-méthyl)]-1,2,3,6-tétrahydropyridine

118,61 mmoles de (3S,8aS)-3-phényl-2,3,8,8a-tétrahydro-[5H]-oxazolo[3,2-a]pyridine dissoutes dans de l'éther anhydre (180 ml) sont additionnées goutte à goutte au réactif de Réformatsky BrZnCH2COOEt (474 ml, 474 mmol) placé à 0°C sous atmosphère d'argon et agitation magnétique. L'addition terminée, on laisse remonter la température à 25°C puis on maintient l'agitation à cette température pendant une nuit. Le mélange réactionnel est versé sur une solution saturée de chlorure d'ammonium à 0°C puis filtré sur büchner afin d'éliminer les précipités insolubles. La phase organique est extraite à l'éther puis à l'acétate d'éthyle et enfin séchée sur sulfate de magnésium. Après évaporation du solvant sous pression réduite, le mélange des tétrahydropyridines est obtenu dans les proportions 60/40 respectivement (déterminées par chromatographie en phase gazeuse après acétylation du brut réactionnel). Les deux diastéréoisomères sont séparés par chromatographie sur gel de silice éluée par un gradient de 400 ml d'acétate d'éthyle/heptane (0/100 à 50/50 de 5 % en 5 %).
*Huile jaune orangé*
*HR (IC) (Méthane) : C*_{*17*}*H*_{*24*}*NO*_{*3*}
*Calculé : 290,1756*
*Trouvé : 290,1769*

### Stade C : (1'S,2"S)-(1-{2-[Diméthyl-(1,1,2-triméthylpropyl)-silanyloxy]-1-phényl-éthyl}-2-{éthoxycarbonyl-méthyl})-1,2,3,6-tétrahydropyridine

30,65 mmoles de (1'S,2"S)-[1-(2-hydroxy-1-phényl-éthyl)-2-(éthoxycarbonyl-méthyl)]-1,2,3,6-tétrahydropyridine sont diluées dans du dichlorométhane anhydre (70 ml) puis placées à 0°C sous atmosphère d'argon. Le diméthylthexylchlorosilane (1,5 eq, 9,0 ml, 45,98 mmol) est additionné puis la triéthylamine (2,5 eq, 10,8 ml, 76,64 mmol) ainsi qu'une quantité catalytique de diméthylaminopyridine. On laisse la température remonter à 25°C puis on maintient l'agitation à cette température pendant une nuit. Le mélange réactionnel est versé sur une solution saturée de chlorure d'ammonium à 0°C. La phase organique est extraite au dichlorométhane puis séchée sur sulfate de magnésium. Après évaporation du solvant sous pression réduite, le brut réactionnel est filtré sur une courte colonne d'alumine (140 g) éluée par un gradient d'acétate d'éthyle/heptane (0/100 à 30/70 de 10 % en 10 %). Le dérivé mono-alkylé silylé est ainsi isolé.
*Huile jaune*
*[α]*_{*D*}*: + 24 (c = 1,7, CHCl*_{*3*}*)*

### Stade D : (1'S,2"S)-2-(1-{2-[Diméthyl-(1,1,2-triméthylpropyl)-silanyloxy]-1-phényl-éthyl}-1,2,3,6-tétrahydropyridin-2-yl)éthanol

27,45 mmoles de (1'S,2"S)-(1-{2-[diméthyl-(1,1,2-triméthylpropyl)-silanyloxy]-1-phényl-éthyl}-2-{éthoxycarbonyl-méthyl})-1,2,3,6-tétrahydropyridine sont diluées dans du THF anhydre (90 ml) et placées à 0°C sous agitation magnétique. On additionne lentement l'hydrure de lithium et d'aluminium (1,5 eq, 2,56 g, 41,17 mmol). On laisse la température remonter à 25°C et l'agitation pendant une nuit. Quand la réaction est totale, on ajoute de l'acétate d'éthyle puis quelques gouttes d'eau pour détruire l'excès d'hydrure. La solution obtenue est filtrée sur célite, rincée à l'acétate d'éthyle puis au méthanol. Le filtrat est séché sur sulfate de magnésium et évaporé sous pression réduite. Le brut réactionnel est ensuite purifié par filtration sur une courte colonne d'alumine (110 g) éluée par un gradient d'acétate d'éthyle/heptane (0/100 à 20/80 de 5 % en 5 %). Le mono-alkylé silylé réduit est ainsi isolé.
*Huile jaune pâle*
*HR (IC) (Méthane) : C*_{*23*}*H*_{*40*}*NO*_{*2*}*Si*
*Calculé : 390,2828*
*Trouvé : 390,2803*
*[α]*_{*D*} *: - 11 (c = 0,65, CHCl*_{*3*}*)*

### Stade E : (1'S,2"S)-2-(1-{2-[Diméthyl-(1,1,2-triméthylpropyl)-silanyloxy]-1-phényl-éthyl}-1,2,3,6-tétrahydropyridin-2-yl)-éthanal

Le chlorure d'oxalyle (4 eq, 8,40 ml, 97,56 mmol) en solution dans du dichlorométhane anhydre (180 ml) est refroidi à -78°C. On ajoute, sous agitation et sous atmosphère d'argon, le diméthylsulfoxyde (6 eq, 10,40 ml, 146,34 mmol) dilué au dichlorométhane anhydre (180 ml). Après 10 minutes, on additionne goutte à goutte 24,39 mmoles de (1'S,2"S)-2-(1-{2-Diméthyl-(1,1,2-triméthylpropyl)-silanyloxy]-1-phényl-éthyl}-1,2,3,6-tétrahydropyridin-2-yl)-éthanol dissoutes dans le dichlorométane anhydre (30 ml). L'agitation magnétique est maintenue à-78°C pendant 15 minutes. On ajoute alors la triéthylamine (8 eq, 27,5 ml, 195,12 mmol) et on laisse le mélange réactionnel remonter à température ambiante. Après addition d'eau, la phase organique est extraite au dichlorométhane, séchée sur sulfate de magnésium et évaporée sous pression réduite. L'aldéhyde obtenu est directement mis en réaction, compte tenu de son instabilité.

### Stade F : (1'S,2"S)-2-{2-[Diméthyl-(1,1,2-triméthylpropyl)-silanyloxy]-1-phényl-éthyl}-1,2,3,6-tétrahydropyridin-2-yl)-1-phényl-éthanol

Dans un tricol, on verse le bromure de phénylmagnésium (1,5 eq, 61 ml, 36,59 mmol) sous atmosphère d'argon et agitation magnétique à 0°C. Le produit brut de la réaction de Swern (9,44 g, 24,39 mmol) dilué dans de l'éther anhydre (80 ml) est additionné goutte à goutte par l'intermédiaire d'une ampoule de brome. L'addition terminée, on laisse remonter la température à 25°C puis on maintient l'agitation à cette température pendant une nuit. Le mélange réactionnel est alors versé sur une solution saturée de chlorure d'ammonium à 0°C. La phase organique est extraite à l'éther puis au dichlorométhane et enfin séchée sur sulfate de magnésium. Après évaporation du solvant sous pression réduite, le mélange des alcools est obtenu dans les proportions 60/40 respectivement (déterminées par intégration des protons en RMN). Les deux diastéréoisomères sont séparés par chromatographie sur alumine (360 g) éluée par un gradient de 400 ml d'acétate d'éthyle/heptane (0/100 à 5/95 de 1 % en 1 % puis 5/95 à 20/80 de 5 % en 5 %). Le diastéréoisomère majoritaire est isolé.
*Pâte brune*
*HR (IC) (Méthane) : C*_{*29*}*H*_{*44*}*NO*_{*2*}*Si*
*Calculé : 466,3141*
*Trouvé : 466,3152*
*[α]*_{*D*}*: - 18 (c = 0,22, CHCl*_{*3*}*)*

### Stade G : (1S,1'S,2"S)-2-[1-(2-Hydroxy-1-phényl-éthyl)-1,2,3,6-tétrahydro-pyridin-2-yl]-1-phényl-éthanol

9,81 mmoles de (1'S,2"S)-2-(1-{2-[Diméthyl-(1,1,2-triméthylpropyl)-silanyloxy]-1-phényl-éthyl}-1,2,3,6-tétrahydropyridin-2-yl)-1-phényl-éthanol sont diluées dans un mélange d'acide chlorhydriqueleau/tétrahydrofurane dans les proportions 3/1/1. L'ensemble est laissé une nuit sous agitation magnétique à température ambiante. Le milieu réactionnel est alors extrait en présence de carbonate de potassium, à l'éther puis au dichlorométhane. La phase organique est séchée sur sulfate de magnésium et évaporée sous pression réduite. Le brut réactionnel est ensuite filtré sur une courte colonne d'alumine (95 g) éluée par un gradient d'acétate d'éthyle/heptane (0/100 à 100/0 de 10 % en 10 %) puis de méthanol/dichlorométhane (0/100 à 5/95 de 1 % en 1 %). Le diol est ainsi isolé.
*Huile jaune*
*HR (IC) (Méthane) : C*_{*21*}*H*_{*26*}*NO*_{*2*}
*Calculé : 324,1964*
*Trouvé : 324,1965*
*[α]*_{*D*} *: - 49 (c = 1,1, CHCl*_{*3*}*)*

### EXEMPLE 2 : (1R,1'S,2"S)-2-[1-(2-Hydroxy-1-phényl-éthyl)-1,2,3,6-tétrahydropyridin-2-yl]-1-phényl-éthanol

On utilise le même procédé que pour l'exemple 1 en choisissant le diastéréoisomère minoritaire au stade F.
*Huile jaune*
*HR (IC) (Méthane) : C*_{*21*}*H*_{*26*}*NO*_{*2*}
*Calculé : 324,1963*
*Trouvé : 324,1941*
*[α]*_{*D*} *: + 52 (c = 2,4, CHCl*_{*3*}*)*

Les exemples 3 à 6 sont obtenus en utilisant le même procédé que pour l'exemple 1 en prenant le magnésien approprié au stade F.

### EXEMPLE 3 : (1R,1'S,2"S)-1-[1-(2-Hydroxy-1-phényl-éthyl)-1,2,3,6-tétrahydropyridin-2-yl]-propanol-2

### EXEMPLE 4 : (1R,1'S,2"S)-1-[1-(2-Hydroxy-1-phényl-éthyl)-1,2,3,6-tétrahydro-pyridin-2-yl]-4-méthyl-pentanol-2

### EXEMPLE 5 : (1S,1'S,2"S)-2-[1-(2-Hydroxy-1-phényl-éthyl)-1,2,3,6-tétrahydro-pyridin-2-yl]-1-(4-méthyl-phényl)-éthanol

### EXEMPLE 6 : (1S,1'S,2"S)-2-[1-(2-Hydroxy-1-phényl-éthyl)-1,2,3,6-tétrahydropyridin-2-yl]-1-(3-trifluorométhyl-phényl)-éthanol

### EXEMPLE 7 : (1S,2"S)-2-(1-Méthyl-1,2,3,6-tétrahydropyridin-2-yl)-1-phényl-éthanol

### Stade A : (1S,2"S)-2-[1-Méthyl-1-(2-hydroxy-1-phényl-éthyl)-1,2,3,6-tétrahydro pyridinium-2-yl]-1-phényl-éthanol-tétrafluoroborate

15,3 mmoles du composé de l'exemple 1 sont dissous dans l'acétonitrile distillé (160 ml). On additionne sous agitation magnétique le diphénylméthyl sulfonium tétrafluoroborate (1,1 eq, 6,67 g, 21,09 mmol) et on porte l'ensemble à reflux pendant 48 heures. Après évaporation du solvant sous pression réduite, le brut réactionnel est chromatographié sur alumine (195 g) éluée par un gradient de 200 ml d'acétate d'éthyle/heptane (0/100 à 50/50 de 10 % en 10 %) afin d'éliminer le diphényl sulfure et le produit n'ayant pas réagi, puis par un gradient de 100 ml de méthanol/dichlorométhane (0/100 à 5/95 de 0,5 % en 0,5 % puis de 5/95 à 50/50 de 5 % en 5 %). On isole ainsi le produit méthylé.
*Mousse beige clair*

### Stade B : (1S,2"S)-2-(1-Méthyl-1,2,3,6-tétrahydropyridin-2-yl)-1-phényl-éthanol

3,56 mmoles du sel obtenu dans le stade A sont dissous dans le tert-butanol distillé (40 ml). On additionne le tert-butanolate de potassium (1,5 eq, 600 mg, 5,34 mmol) préalablement sublimé (145°C, P = 0,6 mm Hg). L'ensemble est porté à reflux pendant 2 heures. Le mélange réactionnel refroidi est versé sur une solution saturée de chlorure d'ammonium, extrait au dichlorométhane et séché sur sulfate de magnésium. La manipulation est reproduite ainsi 3 fois. Après évaporation du solvant sous pression réduite, le brut est chromatographié sur alumine (70 g) élué par un gradient d'acétate d'éthyle/heptane (0/100 à 50/50 de 10 % en 10 %) puis méthanol/dichlorométhane (0/100 à 6/94 de 0,5 % en 0,5 %).
*Huile jaune orangé*
*HR (IE) : C*_{*14*}*H*_{*19*}*NO*
*Calculé : 217,1467*
*Trouvé : 217,1464*

### EXEMPLE 8 : (1R,2"S)-2-(1-Méthyl-1,2,3,6-tétrahydropyridin-2-yl)-1-phényl-éthanol

On procède comme pour l'exemple 7 à partir du composé de l'exemple 2.
*Huile jaune orangé*
*HR (IE) : C*_{*14*}*H*_{*19*}*NO*
*Calculé : 217,1467*
*Trouvé : 217,1466*

### EXEMPLE 9 : (2"S)-2-Benzoylméthyl-1-méthyl-1,2,3,6-tétrahydropyridine

On procède comme dans le stade E de l'exemple 1 à partir du composé de l'exemple 7 ou 8.

### EXEMPLE 10 : (1R,2"S)-1-[1-Méthyl-1,2,3,6-tétrahydropyridin-2-yl]-propanol-2

On procède comme pour l'exemple 7 à partir du composé de l'exemple 3.

### EXEMPLE 11 : (1S,2"S)-2-[1-Méthyl-1,2,3,6-tétrahydropyridin-2-yl]-1-(4-méthyl-phényl)-éthanol

On procède comme pour l'exemple 7 à partir du composé de l'exemple 5.

### EXEMPLE 12 : (2"S)-2-(4-Méthylbenzoylméthyl)-1-méthyl-1,2,3,6-tétrahydro pyridine

On procède comme dans le stade E de l'exemple 1 à partir du composé de l'exemple 11.

### EXEMPLE 13 : (1S,2"S)-2-(1-Méthyl-6-éthoxycarbonylméthyl-1,2,3,6-tétrahydropyridin-2-yl)-1-phényl-éthanol

### Stade A : (1S,2"S)-2-(1-Méthyl-1,2,3,6-tétrahydro-pyridin-2-yl)-1-phényl-éthanol, N-oxyde

1 mmole du composé de l'exemple 7 est diluée dans du dichlorométhane anhydre (10 ml), et placée à 0°C sous agitation magnétique. On additionne l'acide méta-chloroperbenzoïque (1,5 eq, 371 mg, 1,5 mmol). Après 15 minutes, l'ensemble est rapidement filtré sur une courte colonne d'alumine (60 fois le poids théorique) éluée par un gradient de méthanol/dichlorométhane (0/100 à 5/95 de 1 % en 1 %). Après évaporation du solvant sous pression réduite, on isole le N-oxyde qui est immédiatement remis en réacion.

### Stade B : (1S,2"S)-2-(1-méthyl-2,3-dihydropyridinium-2-yl)-1-phényl-éthane trifluoroacétate

1 mmole du N-oxyde obtenu dans le stade A est reprise par du dichlorométhane anhydre (10 ml) et le milieu est placé à 0°C. On additionne lentement l'anhydride trifluoroacétique (2,5 eq, 0,35 ml, 2,5 mmol). La réaction est laissée 20 à 30 minutes à 0°C. Le solvant et le réactif excédentaire sont évaporés sous pression réduite pour fournir le sel de dihydropyridinium. Compte tenu de son instabilité, le composé est directement alkylé.

### Stade C : (1S,2"S)-2-(1-Méthyl-6-éthoxycarbonylméthyl-1,2,3,6-tétrahydropyridin-2-yl)-1-phényl-éthanol

Le sel de dihydropyridinium obtenu au stade B est dilué au dichlorométhane anhydre (10 ml) et additionné goutte à goutte à une solution 1 molaire de réactif de Réformatsky (5 eq, 5 ml, 5 mmol) placée à 0°C sous atmosphère d'argon et agitation magnétique. L'addition terminée, on laisse remonter la température à 25°C puis on maintient l'agitation à cette température pendant une nuit. Le mélange réactionnel est versé sur une solution saturée de chlorure d'ammonium à 0°C. La phase organique est extraite au dichlorométhane puis séchée sur sulfate de magnésium. Après évaporation du solvant sous pression réduite, le brut réactionnel est filtré sur une très courte colonne d'alumine éluée par un mélange d'acétate d'éthyle/heptane (50/50). On obtient alors un mélange inséparable de diastéréoisomères (182 mg, 0,60 mmol) dans les proportions 60/40 (déterminées par intégration des protons en RMN).
*Huile jaune*
*SM (IC) (isobutane) : m/z*
*360 ([M+57]*^{*+*}*,7), 304 ([M+H]*^{*+*}*, 100), 216 ([M-CH*_{*2*}*COOCH*_{*2*}*CH*_{*3*}*]*^{*+*}*,8)*

### EXEMPLE 14 : (1R,2"S)-1-(1-Méthyl-6-éthoxycarbonylméthyl-1,2,3,6-tétrahydropyridin-2-yl)-propanol-2

On procède comme dans l'exemple 13 à partir du composé de l'exemple 10.

### EXEMPLE 15 : (1S,2"S)-2-(1-Méthyl-6-benzoylméthyl-1,2,3,6-tétrahydropyridin-2-yl)-1-phényl-éthanol

### Stade A : (1S,2"S)-2-[6-(2-Hydroxy-2-phényl-éthyl)-1-mélyl-1,2,3,6-tétrahydropyridin-2-yl]-N-méthoxy-N-méthyl acétamide

Le chlorhydrate de N,O-diméthylhydroxylamine (3 eq, 88 mg, 0,90 mmol) dilué au toluène anhydre (1,8 ml) est placé à 0-5°C, sous argon et agitation. On additionne goutte à goutte le triméthylaluminium, solution commerciale à 2 M dans le toluène (1,15 eq, 0,52 ml, 1,04 mmol). L'addition terminée, on retire le bain d'eau glacée et on laisse l'ensemble sous agitation pendant 2 heures. Le réactif de Weinreb ainsi préparé est additionné goutte à goutte sous atmosphère d'argon au composé de l'exemple 13 (91 mg, 0,30 mmol) dilué au toluène anhydre (3 ml). L'ensemble est porté à 80°C, toujours sous argon, pendant 2 h 45. Le brut réactionnel est alors versé doucement sur une solution d'acide chlorhydrique (0,12 N) à 0°C. L'ensemble est laissé sous agitation pendant une à deux minutes puis reversé immédiatement sur une solution de bicarbonate de sodium à 0°C jusqu'à neutralité du milieu réactionnel. La phase organique est alors extraite à l'éther puis cinq fois au dichlorométhane et enfin séchée sur sulfate de magnésium. Après évaporation des solvants sous pression réduite, l'hydroxyamide obtenu (90 mg, 0,28 mmol) est directement remis en réaction.
*SM (IC) (isobutane) : m/z*
*319 ([M+H]*^{*+*}*,100), 216 ([M-CH*_{*2*}*CON(Me)OMe]*^{*+*}*,10)*

### Stade B : (1S,2"S)-2-(1-Méthyl-6-benzoylméthyl-1,2,3,6-tétrahydropyridin-2-yl)-1-phényl-éthanol

0,28 mmoles de l'hydroxyamide obtenue dans le stade A sont placées dans le tétrahydrofurane anhydre (3 ml) refroidi à -78°C sous atmosphère d'argon et agitation magnétique. On additionne goutte à goutte le phényllithium, solution commerciale à 1,6 M dans le mélange cyclohexane/éther (5 eq, 0,88 ml, 1,41 mmol). L'addition terminée, on laisse la température remonter à 25°C puis on maintient l'agitation à cette température pendant une nuit. Le mélange réactionnel est versé sur une solution saturée de chlorure d'ammonium à 0°C. La phase organique est extraite une première fois à l'éther puis au dichlorométhane et enfin séchée sur sulfate de magnésium. Le brut de la déhydrolobéline est directement transformé en son chlorhydrate par traitement au méthanol chlorhydrique. L'ensemble est évaporé, repris dans un minimum de méthanol et lavé trois fois à l'heptane. Le sel est alors rapidement filtré sur silice élué par du méthanol/dichlorométhane (0, 0,5, 1 et 2 %). Le chlorhydrate de la déhydrolobéline est ainsi isolé, sans autre forme de purification. Le traitement du chlorhydrate en milieu basique conduit au mélange inséparable de diastéréoisomères dans les proportions 85/15 (déterminées par intégration des protons en RMN).
*SM (IE) : m/z*
*335 (M*^{*+ •*}*,10), 230 ([M+*^{*•*}*-*^{*•*}*COPh],21), 216 ([M*^{*+•*}*-*^{*•*}*CH*_{*2*}*COPh],100), 105 (PhCO*^{*+*}*,57),*
*94 (PhOH*^{*+•*}*,99)*

### EXEMPLE 16 : (2"S)-2,6-di(Benzoylméthyl)-1-méthyl-1,2,3,6-tétrahydropyridine

On procède comme dans le stade E de l'exemple 1 à partir du composé de l'exemple 15.

### EXEMPLE 17 : (1R,2"S)-1-(1-Méthyl-6-benzoylméthyl-1,2,3,6-tétrahydropyridin-2-yl)-propanol-2

On procède comme pour l'exemple 15 à partir du composé de l'exemple 14.

### EXEMPLE 18 : (2"S)-2-Acétylméthyl-6-benzoylméthyl-1-méthyl-1,2,3,6-tétrahydropyridine

On procède comme dans le stade E de l'exemple 1 à partir du composé de l'exemple 17.

### EXEMPLE 19 : (1S,2"S,6"R)-2-[1-Méthyl-6-(1,1-éthylènedioxyphénéthyl-2)-1,2,3,6-tétrahydropyridin-2-yl]-1-phényl-éthanol

On place dans un petit ballon le benzène (5 ml), l'éthylène glycol en large excès (1 ml) et l'acide paratoluène sulfonique (1,2 eq, 27 mg, 0,143 mmol). On porte l'ensemble à reflux du Dean-Stark pendant 10 minutes environ pour éliminer toute trace d'eau. On ajoute alors le composé de l'exemple 15 sous forme de base (40 mg, 0,119 mmol) diluée dans quelques millilitres de benzène. Après 4 heures au reflux du Dean-Stark, on verse doucement le milieu réactionnel refroidi sur une solution de bicarbonate de sodium. La phase organique est extraite une première fois à l'éther puis au dichlorométhane et enfin séchée sur sulfate de magnésium. Après évaporation des solvants sous pression réduite, l'acétal est rapidement filtré sur alumine élué par du dichlorométhane et isolé.
*Huile jaune*
*SM (IE) : m/z*
*379 (M*^{*+•*}*,10), 258 ([M*^{*+•*}*-*^{*•*}*CH*_{*2*}*CH(OH)Ph],61), 216 ([M*^{*+•*}*-CH*_{*2*}*C(OCH*_{*2*}*)*_{*2*}*Ph],100), 149 (PhC(OCH*_{*2*}*)*_{*2*}^{*+*}*,99)*

### EXEMPLE 20 : (2"S,6"R)-2-Benzoylméthyl-1-méthyl-6-(1,1-éthylènedioxyphénéthyl-2)-1,2,3,6-tétrahydropyridine

On procède comme dans le stade E de l'exemple 1 à partir du composé de l'exemple 19.

### ETUDE PHARMACOLOGIQUE

### EXEMPLE A : Etude de la capacité des composés de l'invention à se lier aux récepteurs nicotiniques

Les composés de la présente invention ont été étudiés en première intention sur leurs capacités à se lier aux récepteurs nicotiniques en utilisant la méthode de Marks et coll. (J. Pharmacol. Exp. Ther., 1986, 30, 427-436). Ils ont montré une affinité intéressante pour le récepteur α7 en utilisant pour radio-ligand 1'(¹²⁵I)-α-bungarotoxine. Cette activité a été confirmée dans la technique des ovocytes de Xenopus injectés par ARNm spécifique des récepteurs α7.

### EXEMPLE B : Test d'évitement passif chez le Rat

L'étude des propriétés facilitatrices de la mémoire a été réalisée sur le test d'évitement passif chez le Rat lors d'amnésie expérimentale par blocage cholinergique.

Ce test a été effectué en utilisant une boite à deux compartiments, un noir non éclairé et un blanc très éclairé par une lampe de 25W. Les deux compartiments sont séparés par une porte à guillotine et le plancher du compartiment noir permet de délivrer un choc électrique (0.5 mA durant 3 secondes). Lors de la phase d'apprentissage, l'animal est placé dans le compartiment blanc et la porte de séparation est ouverte après 60 secondes. Après entrée de l'animal dans le compartiment sombre, la porte est refermée et l'animal reçoit un choc électrique. Lors de la phase de remémoration, 24 heures plus tard, l'animal est placé à nouveau dans le compartiment blanc. Dans ces conditions, un rat normal ne repasse plus dans le sombre puisqu'il se souvient du choc électrique aversif. Le temps maximum de mesure de la latence d'entrée est fixé à 300 secondes. Lors d'amnésie induite par blocage cholinergique, l'animal entre à nouveau dans le compartiment puni.

Lors de traitement par la lobéline ou par les composés de l'invention, les rats ont été protégés de l'amnésie expérimentale et ne sont donc pas repassés dans le compartiment puni ce qui témoigne de l'intégrité des fonctions de mémoire des faits récents.

### EXEMPLE C : Composition pharmaceutique

Formule de préparation pour 1000 comprimés dosés à 10 mg du composé de l'exemple 15 :

| | |
|---|---|
| (1S,2"S)-2-(1-Méthyl-6-benzoylméthyl-1,2,3,6-tétrahydropyridin-2-yl)-1-phényl-éthanol | 10 g |
| Amidon de blé | 15 g |
| Amidon de maïs | 15 g |
| Lactose | 65 g |
| Stéarate de magnésium | 2 g |
| Silice | 1 g |
| Hydroxypropylcellulose | 2 g |

## Revendications

1. Composés de formule (I) 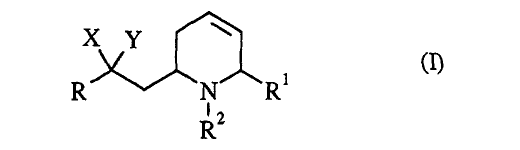 dans laquelle :
- X représente un atome d'hydrogène,
- Y représente un groupement hydroxy,
ou X et Y forment ensemble un groupement oxo,
- R représente un groupement alkyle (C₁-C₆) linéaire ou ramifié ou un groupement phényle éventuellement substitué par un ou plusieurs atomes d'halogène ou un ou plusieurs groupements alkyle (C₁-C₆) linéaire ou ramifié, alkoxy (C₁-C₆), polyhalogéno alkyle (C₁-C₆) linéaire ou ramifié, ou hydroxy,
- R¹ représente un atome d'hydrogène ou un groupement de formule (II) : 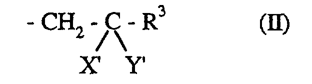 dans laquelle :
X' représente un atome d'hydrogène,
Y' représente un groupement hydroxy,
ou X' et Y' représentent simultanément un groupement alkoxy (C₁-C₆) linéaire ou ramifié,
ou X' et Y' forment ensemble un groupement oxo ou un groupement alkylène dioxy (C₁-C₄) linéaire ou ramifié,
R³ représente un groupement alkoxy (C₁-C₆) linéaire ou ramifié, ou un groupement phényle éventuellement substitué par un ou plusieurs atomes d'halogène, ou un ou plusieurs groupements alkyle (C₁-C₆) linéaire ou ramifié, alkoxy (C₁-C₆), polyhalogénoalkyle (C₁-C₆) linéaire ou ramifié, ou hydroxy,
- R² représente un atome d'hydrogène, un groupement alkyle (C₁-C₆) linéaire ou ramifié, éventuellement substitué par un ou plusieurs groupements, identiques ou différents, aryle éventuellement substitué, alkoxy (C₁-C₆) linéaire ou ramifié, ou hydroxy,
étant entendu que lorsque R et R² représentent simultanément un groupement méthyle, X représente un atome d'hydrogène, Y représente un groupement hydroxy et R¹ représente un groupement de formule (II) où X' représente un atome d'hydrogène et Y' un groupement hydroxy, alors R³ ne peut représenter un groupement phényle,
leurs isomères ainsi que leurs sels d'addition à un acide ou à une base pharmaceutiquement acceptable.

2. Composés de formule (I) selon la revendication 1 pour lesquels X représente un atome d'hydrogène et Y un groupement hydroxy.

3. Composés de formule (I) selon la revendication 2 pour lesquels R¹ représente un groupement de formule (II) :

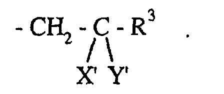

4. Composé de formule (I) selon la revendication 1 qui est le (1S,2"S)-2-(1-Méthyl-6-benzoyl-méthyl-1,2,3,6-tétrahydropyridin-2-yl)-1-phényl-éthanol.

5. Composé de formule (I) selon la revendication 1 qui est le (1S,2"S,6"R)-2-[1-Méthyl-6-(1,1-éthylènedioxyphénéthyl-2)-1,2,3,6-tétrahydropyridin-2-yl]-1-phényl-éthanol.

6. Procédé de préparation des composés de formule (I) selon la revendication 1 **caractérisé en ce que** l'on utilise comme produit de départ un composé de formule (III) : 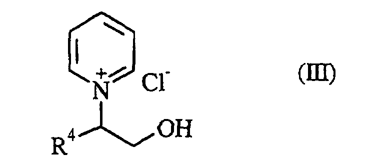 dans laquelle R⁴ représente un groupement alkyle (C₁-C₆) linéaire ou ramifié ou aryle éventuellement substitué,
que l'on fait réagir avec du borohydrure de sodium en présence d'hydroxyde de sodium pour obtenir le composé de formule (IV) : 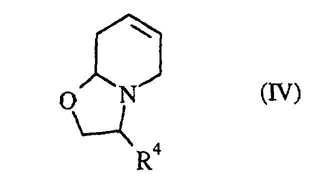 dans laquelle R⁴ est tel que défini précédemment,
qui, soumis à l'action de BrZnCH₂COOEt, conduit au composé de formule (V): 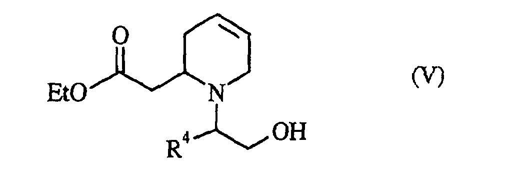 dans laquelle R⁴ a la même définition que précédemment,
qui est successivement soumis à l'action du diméthylthexylchlorosilane puis à celle de l'hydrure de lithium et d'aluminium, pour conduire au composé de formule (VI) : 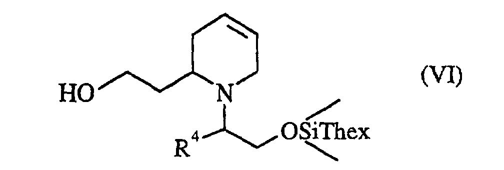 dans laquelle R⁴ est tel que défini précédemment, et Thex représente un groupement (1,1,2)-triméthylpropyl, qui est oxydé pour conduire au composé de formule (VII): 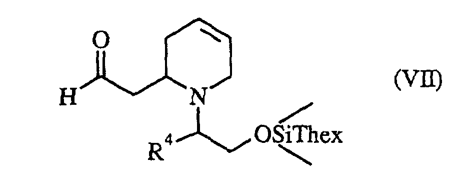 dans laquelle R⁴ et Thex sont définis comme précédemment,
qui est soumis à l'action du magnésien RMgBr où R est tel que défini dans la formule (I), pour conduire au composé de formule (VIII) : 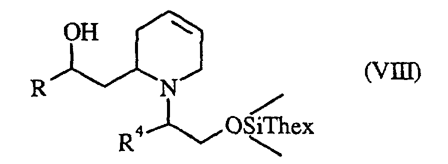 dans laquelle R, R⁴ et Thex sont tels que définis précémment,
qui est éventuellement soumis à un agent oxydant, pour conduire au composé de formule (IX): 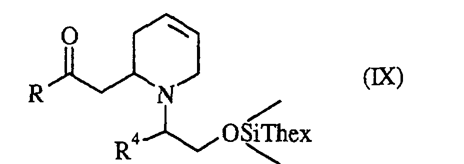 dans laquelle R, R⁴ et Thex ont la même définition que précédemment,
l'ensemble des composés (VIII) et (IX) formant le composé de formule (X) : 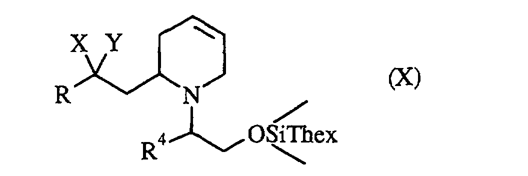 dans laquelle R, R⁴, X, Y et Thex sont tels que définis dans la formule (I) et R⁴ a la même définition que précédemment,
composé de formule (X) qui est hydrolysé en milieu acide, pour conduire au composé de formule (I/a), cas particulier des composés de formule (I) : 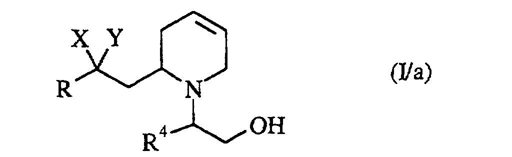 dans laquelle R, X, Y et R⁴ sont tels que définis précédemment,
qui est :
- soit traité, lorsque X représente un atome d'hydrogène et Y un groupement hydroxy, par de l'hydrogène en présence de palladium sur charbon, pour obtenir le composé de formule (I/b), cas particulier des composés de formule (I) : 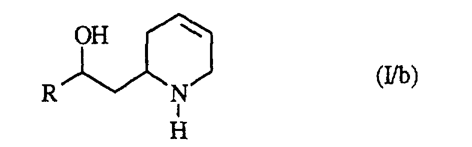 dans laquelle R est tel que défini précédemment,
- soit traité par un diphénylalkylsulfonium tétrafluoroborate de formule (XI): 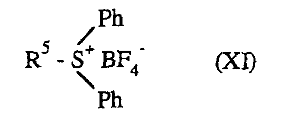
dans laquelle R⁵ représente un groupement alkyle (C₁-C₆) linéaire ou ramifié,
pour aboutir au composé de formule (XII) : 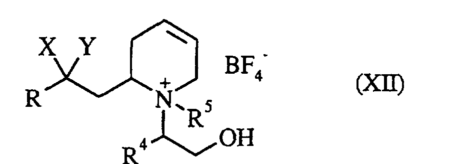 dans laquelle R, R⁴, R⁵, X et Y sont tels que définis précédemment,
qui est soumis à l'action du tertbutanolate de potassium dans du tertbutanol, pour conduire au composé de formule (I/c), cas particulier des composés de formule (I): 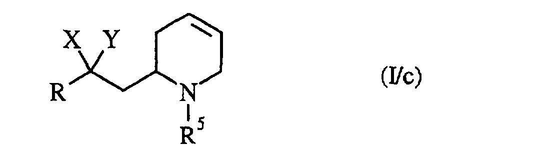 dans laquelle R, X, Y et R⁵ sont tels que définis précédemment,
qui est, dans le cas où X représente un atome d'hydrogène et Y un groupement hydroxy traité par l'acide métachloroperbenzoïque, pour obtenir le composé de formule (XIII) : 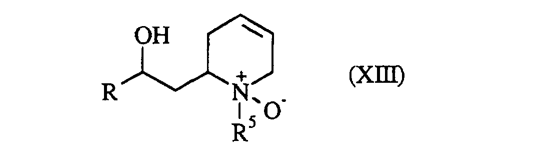 dans laquelle R et R⁵ ont la même définition que précédemment,
qui est soumis à l'action de l'anhydride trifluoroacétique, pour conduire au composé de formule (XIV) : 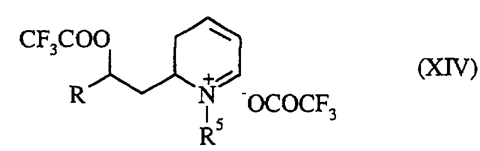 dans laquelle R et R⁵ sont tels que définis précédemment,
que l'on traite par BrZnCH₂COOR⁶ où R⁶ représente un groupement alkyle (C₁-C₆) linéaire ou ramifié, pour obtenir le composé de formule (I/d), cas particulier des composés de formule (I): 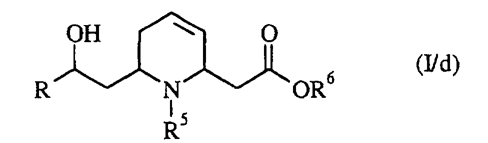 dans laquelle R, R⁵ et R⁶ ont la même définition que précédemment,
qui est successivement soumis au réactif de Weinreb 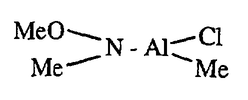 puis à l'action d'un lithien R⁷-Li où R⁷ représente un groupement phényle éventuellement substitué par un ou plusieurs atomes d'halogène ou un ou plusieurs groupements alkyle (C₁-C₆) linéaire ou ramifié, alkoxy (C₁-C₆), polyhalogénoalkyle (C₁-C₆) linéaire ou ramifié, hydroxy, pour conduire au composé de formule (I/e), cas particulier des composés de formule (I) : 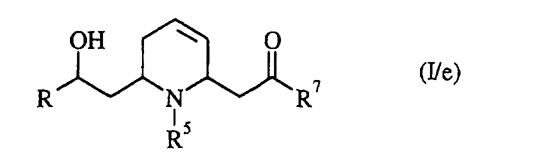 dans laquelle R, R⁵ et R⁷ sont tels que définis précédemment,
qui est :
- soit soumis à un agent oxydant, pour conduire au composé de formule (I/f), cas particulier des composés de formule (I) : 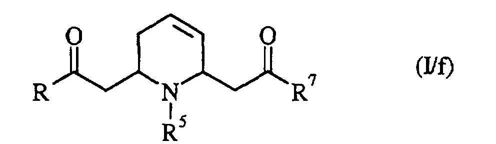 dans laquelle R, R⁵ et R⁷ ont la même définition que précédemment,
- soit soumis à un agent réducteur, pour conduire au composé de formule (I/g), cas particulier des composés de formule (I): 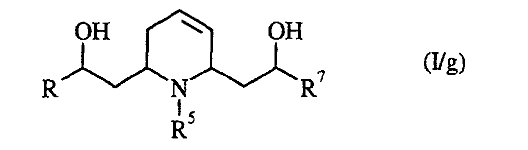 dans laquelle R, R⁵ et R⁷ sont tels que définis précédemment,
- soit protégés sous forme d'acétal par l'action d'un alcool en milieu acide, pour obtenir le composé de formule (I/h), cas particulier des composés de formule (I) : 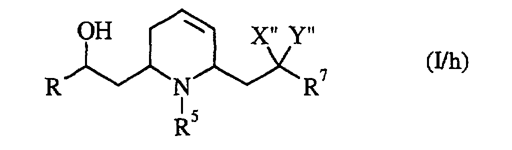
dans laquelle R, R⁵ et R⁷ sont tels que définis précédemment, et X" et Y" représentent simultanément un groupement alkoxy (C₁-C₆) linéaire ou ramifié ou forment ensemble un groupement alkylènedioxy (C₁-C₄) linéaire ou ramifié,
les composés (I/a) à (I/h) formant l'ensemble des composés de l'invention que l'on purifie, le cas échéant, selon une technique classique de purification, qui peuvent, si on le désire, être séparés en leurs différents isomères optiques ou salifiés avec un acide ou une base pharmaceutiquement acceptable.

7. Composés de formule (V), (XII) et (XIII) selon la revendication 6 utiles comme intermédiaires de synthèse des composés de formule (I) selon la revendication 1.

8. Compositions pharmaceutiques contenant au moins un composé de formule (I) selon les revendications 1 à 5 ou un de leurs sels d'addition à un acide pharmaceutiquement acceptable en combinaison avec un ou plusieurs excipients ou véhicules inertes, non toxiques, pharmaceutiquement acceptable.

9. Compositions pharmaceutiques selon la revendication 8 contenant au moins un principe actif, selon l'une quelconque des revendications 1 à 5, utiles pour le traitement des déficits de mémoire liés au vieillissement cérébral et aux maladies neurodégénératives telles que la maladie d'Alzheimer, la maladie de Parkinson, la maladie de Pick, la maladie de Korsakoff et les démences frontales et sous-corticales.

## Patentansprüche

1. Verbindungen der Formel (I): 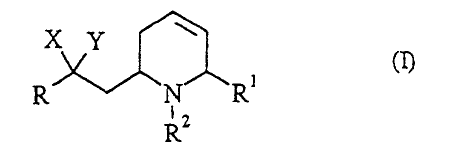 in der
- X ein Wasserstoffatom bedeutet,
- Y eine Hydroxygruppe darstellt,
oder X und Y gemeinsam eine Oxogruppe bilden,
- R eine geradkettige oder verzweigte (C₁-C₆)-Alkylgruppe oder eine Phenylgruppe, die gegebenenfalls durch ein oder mehrere Halogenatome oder ein oder mehrere geradkettige oder verzweigte (C₁-C₆)-Alkylgruppen, (C₁-C₆)-Alkoxygruppen, geradkettige oder verzweigte (C₁-C₆)-Polyhalogenalkylgruppen oder Hydroxygruppen substituiert ist, bedeutet,
- R¹ ein Wasserstoffatom oder eine Gruppe der Formel (II) darstellt: 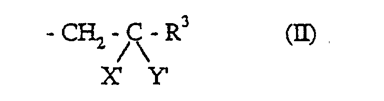 in der:
X' ein Wasserstoffatom darstellt,
Y' eine Hydroxygruppe darstellt,
oder X' und Y' gleichzeitig eine geradkettige oder verzweigte (C₁-C₆)-Alkoxygruppe darstellen,
oder X' und Y' gemeinsam eine Oxogruppe oder eine geradkettige oder verzweigte (C₁-C₄)-Alkylendioxygruppe bilden und
R³ eine geradkettige oder verzweigte (C₁-C₆)-Alkoxygruppe oder eine Phenylgruppe, die gegebenenfalls durch ein oder mehrere Halogenatome oder ein oder mehrere geradkettige oder verzweigte (C₁-C₆)-Alkylgruppen, (C₁-C₆)-Alkoxygruppen, geradkettige oder verzweigte (C₁-C₆)-Polyhalogenalkylgruppen oder Hydroxygruppen substituiert ist, darstellt,
- R² ein Wasserstoffatom, eine geradkettige oder verzweigte, gegebenenfalls durch ein oder mehrere gleichartige oder verschiedene Gruppen substituierte (C₁-C₆)-Alkylgruppe, eine gegebenenfalls substituierte Arylgruppe, eine geradkettige oder verzweigte (C₁-C₆)-Alkoxygruppe oder eine Hydroxygruppe bedeutet,
mit der Maßgabe, daß wenn R und R² gleichzeitig eine Methylgruppe, X ein Wasserstoffatom, Y eine Hydroxygruppe und R¹ eine Gruppe der Formel (II), in der X' ein Wasserstoffatom und Y' eine Hydroxygruppe darstellen, bedeuten, R³ keine Phenylgruppe bedeutet,
deren Isomere sowie deren Additionssalze mit einer pharmazeutisch annehmbaren Säure oder Base.

2. Verbindungen der Formel (I) nach Anspruch 1, worin X ein Wasserstoffatom und Y eine Hydroxygruppe bedeuten.

3. Verbindungen der Formel (I) nach Anspruch 2, worin R¹ eine Gruppe der Formel (II): 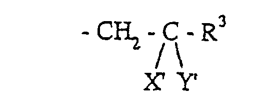 darstellt.

4. Verbindung der Formel (I) nach Anspruch 1, nämlich (1S,2"S)-2-(1-Methyl-6-benzoyl-methyl-1,2,3,6-tetrahydropyridin-2-yl)-1-phenyl-ethanol.

5. Verbindung der Formel (I) nach Anspruch 1, nämlich (1S,2"S,6"R)-2-[1-Methyl-6-( 1,1-ethylendioxyphenethyl-2)-1,2,3,6-tetrahydropyridin-2-yl]-1-phenylethanol.

6. Verfahren zur Herstellung der Verbindungen der Formel (I) nach Anspruch 1, **dadurch gekennzeichnet, daß** man als Ausgangsprodukt eine Verbindung der Formel (III) verwendet: 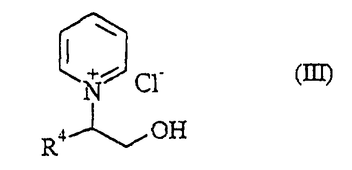 in der R⁴ eine geradkettige oder verzweigte (C₁-C₆)-Alkylgruppe oder eine gegebenenfalls substituierte Arylgruppe bedeutet,
welche man mit Natriumborhydrid in Gegenwart von Natriumhydroxid umsetzt zur Bildung der Verbindung der Formel (IV): 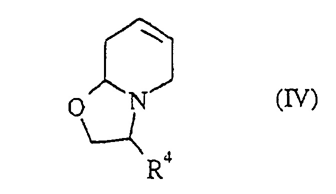 in der R⁴ die oben angegebenen Bedeutungen besitzt,
welche unter der Einwirkung von BrZnCH₂COOEt zu der Verbindung der Formel (V) führt: 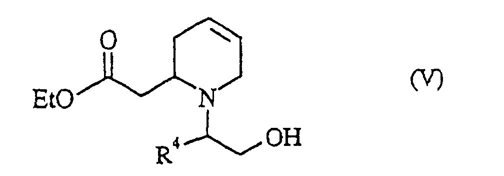 in der R⁴ die oben angegebenen Bedeutungen besitzt,
welche nacheinander der Einwirkung von Dimethylhexylchlorsilan und dann der von Lithiumaluminiumhydrid unterworfen wird zur Bildung der Verbindung der Formel (VI): 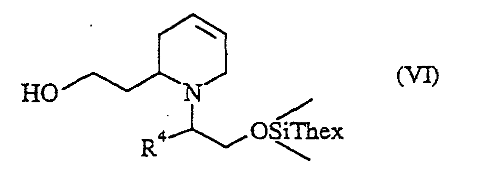 in der R⁴ die oben angegebenen Bedeutungen besitzt und Thex eine (1,1,2)-Trimethylpropylgruppe bedeutet, welche Verbindung oxidiert wird zur Bildung der Verbindung der Formel (VII): 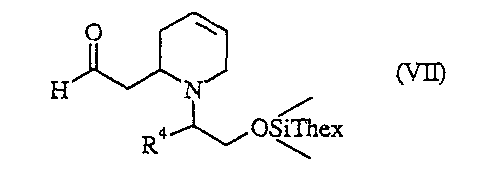 in der R⁴ und Thex die oben angegebenen Bedeutungen besitzen,
welche mit der Magnesiumverbindung RMgBr, worin R die bezüglich der Forme (I) angegebenen Bedeutungen besitzt, unterworfen wird
zur Bildung der Verbindung der Formel (VIII): 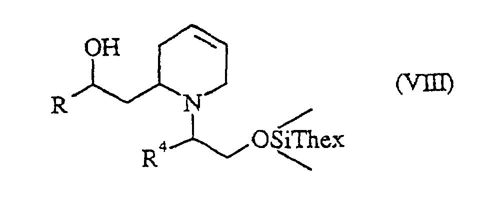 in der R, R⁴ und Thex die oben angegebenen Bedeutungen besitzen,
welche gegebenenfalls der Einwirkung eines Oxidationsmittels unterworfen wird zur Bildung der Verbindung der Formel (IX): 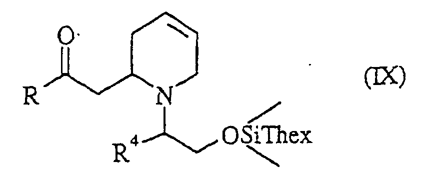 in der R, R⁴ und Thex die oben angegebenen Bedeutungen besitzen,
wobei die Gesamtheit der Verbindungen (VIII) und (IX) die Verbindungen der Formel (X) bilden: 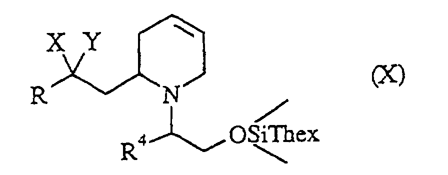 in der R, R⁴, X, Y und Thex die bezüglich der Formel (I) angegebenen Bedeutungen besitzen und R⁴ die oben angegebenen Bedeutungen besitzt,
welche Verbindung der Formel (X) in saurem Medium hydrolysiert wird zur Bildung der Verbindung der Formel (I/a), einem Sonderfall der Verbindungen der Formel (I): 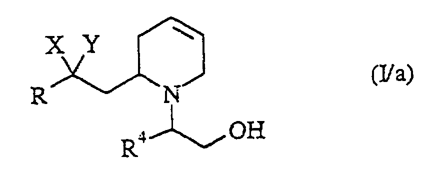 in der R, X, Y und R⁴ die oben angegebenen Bedeutungen besitzen,
welche:
- entweder, wenn X ein Wasserstoffatom und Y eine Hydroxygruppe bedeuten, mit Wasserstoff in Gegenwart von Palladium-auf-Kohlenstoff behandelt wird zur Bildung der Verbindungen der Formel (I/b), einem Sonderfall der Verbindungen der Formel (I): 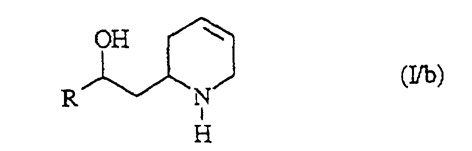 in der R die oben angegebenen Bedeutungen besitzt,
- oder mit einem Diphenylalkylsulfoniumtetrafluorborat der Formel (XI): 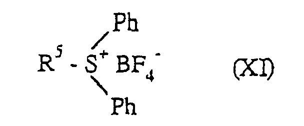 in der R⁵ eine geradkettige oder verzweigte (C₁-C₆)-Alkylgruppe darstellt, behandelt wird zur Bildung der Verbindung der Formel (XII): 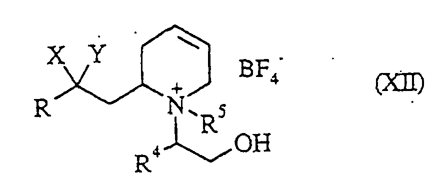 in der R, R⁴, R⁵, X und Y die oben angegebenen Bedeutungen besitzen,
welche der Einwirkung von Kaliumtert.-butanolat in tert.-Butanol unterworfen wird zur Bildung der Verbindung der Formel (I/c), einem Sonderfall der Verbindungen der Formel (I): 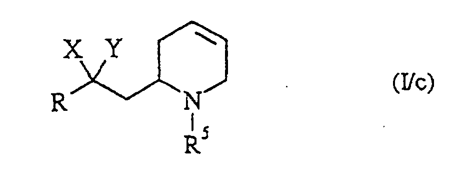 in der R, X, Y und R⁵ die oben angegebenen Bedeutungen besitzen,
welche dann, wenn X ein Wasserstoffatom und Y eine Hydroxygruppe bedeuten, mit m-Chlorperbenzoesäure behandelt wird zur Bildung der Verbindung der Formel (XIII): 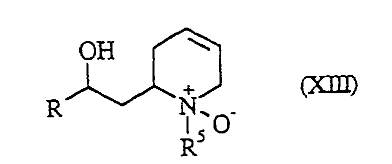 in der R und R⁵ die oben angegebenen Bedeutungen besitzen,
welche der Einwirkung von Trifluoressigsäureanhydrid unterworfen wird zur Bildung der Verbindung der Formel (XIV): 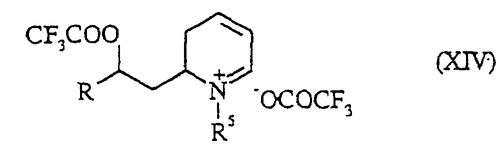 in der R und R⁵ die oben angegebenen Bedeutungen besitzen,
welche man mit BrZnCH₂COOR⁶, in der R⁶ eine geradkettige oder verzweigte (C₁-C₆)-Alkylgruppe darstellt, behandelt zur Bildung der Verbindung der Formel (I/d), einem Sonderfall der Verbindungen der Formel (I): 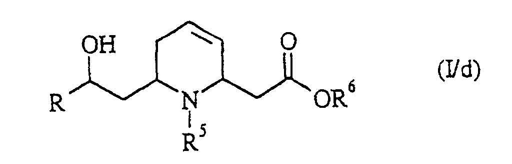 in der R, R⁵ und R⁶ die oben angegebenen Bedeutungen besitzen,
welche nacheinander mit dem Weinreb-Reagens 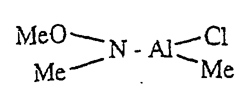 behandelt wird und dann der Einwirkung einer Lithiumverbindung R⁷-Li, worin R⁷ eine Phenylgruppe darstellt, die gegebenenfalls durch ein oder mehrere Halogenatome oder eine oder mehrere geradkettige oder verzweigte (C₁-C₆)-Alkylgruppen, (C₁-C₆)-Alkoxygruppen, geradkettige oder verzweigte (C₁-C₆)-Polyhalogenalkylgruppen oder Hydroxygruppen substituiert ist, unterworfen wird zur Bildung der Verbindung der Formel (I/e), einem Sonderfall der Verbindungen der Formel (I): 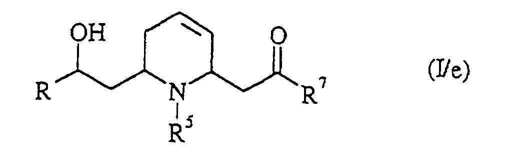 in der R, R⁵ und R⁷ die oben angegebenen Bedeutungen besitzen, welche:
- entweder der Einwirkung eines Oxidationsmittels unterworfen wird zur Bildung der Verbindung der Formel (I/f), einem Sonderfall der Verbindungen der Formel (I): 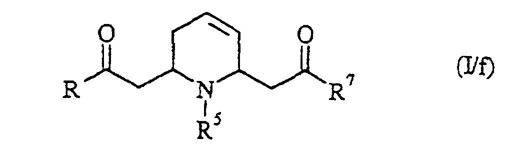 in der R, R⁵ und R⁷ die oben angegebenen Bedeutungen besitzen,
- oder der Einwirkung eines Reduktionsmittels unterworfen wird zur Bildung der Verbindung der Formel (I/g), einem Sonderfall der Verbindungen der Formel (I): 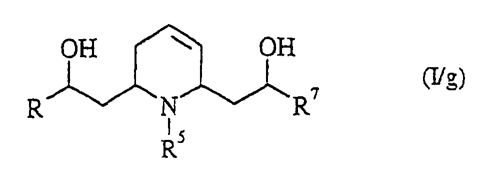 in der R, R⁵ und R⁷ die oben angegebenen Bedeutungen besitzen,
- oder durch Einwirkung eines Alkohols in saurem Medium in Form des Acetals geschützt wird, zur Bildung der Verbindung der Formel (I/h), einem Sonderfall der Verbindungen der Formel (I): 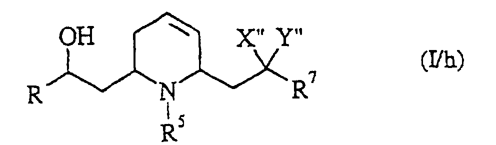 in der R, R⁵ und R⁷ die oben angegebenen Bedeutungen besitzen und X" und Y" gleichzeitig eine geradkettige oder verzweigte (C₁-C₆)-Alkoxygruppe bedeuten oder gemeinsam eine geradkettige oder verzweigte (C₁-C₄)-Alkylendioxygruppe bilden,
welche Verbindungen der Formeln (I/a) bis (I/h) die Gesamtheit der Verbindungen der vorliegenden Erfindung bilden, welche man gegebenenfalls mit Hilfe einer klassischen Reinigungsmethode reinigt, welche gewünschtenfalls in ihre verschiedenen optischen Isomeren aufgetrennt werden oder mit einer pharmazeutisch annehmbaren Säure oder Base in ihre Salze überführt werden können.

7. Verbindungen der Formeln (V), (XII) und (XIII) nach Anspruch 6 als Zwischenprodukte für die Synthese der Verbindungen der Formel (I) nach Anspruch 1.

8. Pharmazeutische Zubereitungen enthaltend mindestens eine Verbindung der Formel (I) nach den Ansprüchen 1 bis 5 oder eines ihrer Additionssalze mit einer pharmazeutisch annehmbaren Säure in Kombination mit einem oder mehreren inerten, nichttoxischen, pharmazeutisch annehmbaren Trägermaterialien oder Bindemitteln.

9. Pharmazeutische Zubereitungen nach Anspruch 8 enthaltend mindestens einen Wirkstoff nach einem der Ansprüche 1 bis 5 zur Behandlung von Gedächtnisstörungen, die mit dem Altern des Gehirns und mit neurodegenerativen Erkrankungen verknüpft sind, wie der Alzheimerschen Krankheit, der Parkinsonschen Krankheit, der Pickschen Krankheit, der Korsakoffschen Krankheit und frontalen und subkortikalen Demenzien.

## Claims

1. Compounds of formula (I): 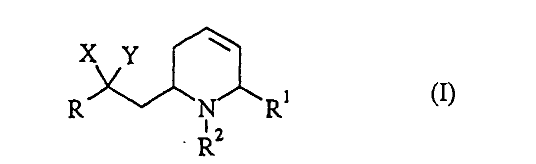 in which:
- X represents a hydrogen atom,
- Y represents a hydroxy group,
or X and Y together form an oxo group,
- R represents a linear or branched (C₁-C₆)alkyl group or a phenyl group optionally substituted by one or more halogen atoms or one or more linear or branched (C₁-C₆)alkyl groups, (C₁-C₆)alkoxy groups, linear or branched (C₁-C₆)polyhaloalkyl groups, or hydroxy groups,
- R¹ represents a hydrogen atom or a group of formula (II): 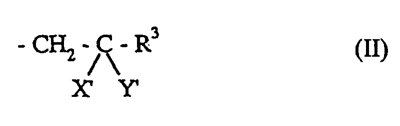 in which:
X' represents a hydrogen atom,
Y' represents a hydroxy group,
or X' and Y' simultaneously represent a linear or branched (C₁-C₆)alkoxy group,
or X' and Y' together form an oxo group or a linear or branched (C₁-C₄)alkylenedioxy group,
R³ represents a linear or branched (C₁-C₆)alkoxy group, or a phenyl group optionally substituted by one or more halogen atoms or one or more linear or branched (C₁-C₆)-alkyl groups, (C₁-C₆)alkoxy groups, linear or branched (C₁-C₆)polyhaloalkyl groups, or hydroxy groups,
- R² represents a hydrogen atom, a linear or branched (C₁-C₆)alkyl group optionally substituted by one or more identical or different optionally substituted aryl groups, linear or branched (C₁-C₆)alkoxy groups, or hydroxy groups,
wherein R³ may not represent a phenyl group when R and R² simultaneously represent a methyl group, X represents a hydrogen atom, Y represents a hydroxy group and R¹ represents a group of formula (II) wherein X' represents a hydrogen atom and Y' represents a hydroxy group,
their isomers, and addition salts thereof with a pharmaceutically acceptable acid or base.

2. Compounds of formula (I) according to claim 1 in which X represents a hydrogen atom and Y represents a hydroxy group.

3. Compounds of formula (I) according to claim 2 in which R¹ represents a group of formula (II):

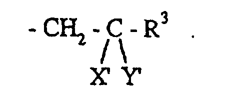

4. Compound of formula (I) according to claim 1 which is (1S,2"S)-2-(1-methyl-6-benzoyl-methyl-1,2,3,6-tetrahydropyridin-2-yl)-1-phenyl-ethanol.

5. Compound of formula (I) according to claim 1 which is (1S,2"S,6"R)-2-[1-methyl-6-(1,1-ethylenedioxyphenethyl-2)-1,2,3,6-tetrahydropyridin-2-yl]-1-phenyl-ethanol.

6. Process for the preparation of compounds of formula (I) according to claim 1, which process is **characterised in that** there is used as starting material a compound of formula (III): 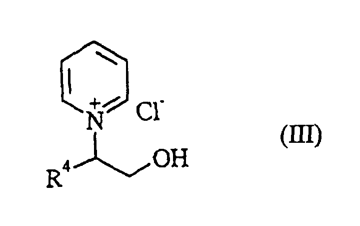 in which R⁴ represents an optionally substituted aryl or linear or branched (C₁-C₆)-alkyl group,
which is reacted with sodium borohydride in the presence of sodium hydroxide to give a compound of formula (IV): 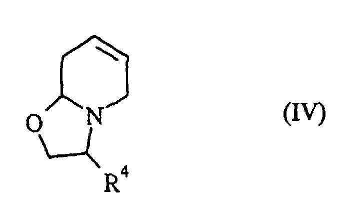 in which R⁴ is as defined above,
which is subjected to the action of BrZnCH₂COOEt to yield a compound of formula (V): 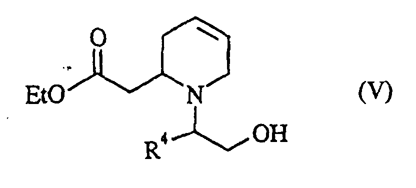 in which R⁴ is as defined above,
which is subjected in succession to the action of dimethylthexylchlorosilane and then to the action of lithium aluminium hydride to yield a compound of formula (VI): 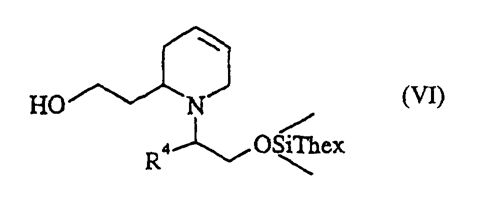 in which R⁴ is as defined above and Thex represents a (1,1,2)-trimethylpropyl group, which is oxidised to yield a compound of formula (VII): 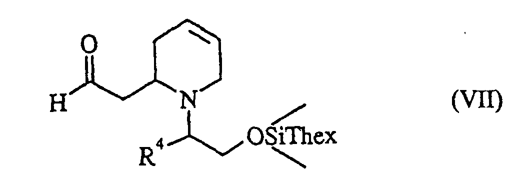 in which R⁴ and Thex are as defined above,
which is subjected to the action of a magnesium compound RMgBr, wherein R is as defined in formula (I), to yield a compound of formula (VIII): 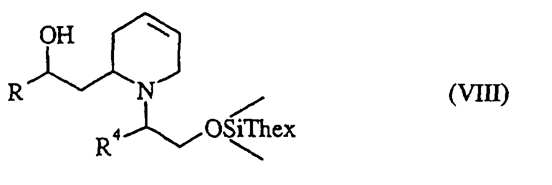 in which R, R⁴ and Thex are as defined above,
which is optionally subjected to an oxidising agent to yield a compound of formula (IX): 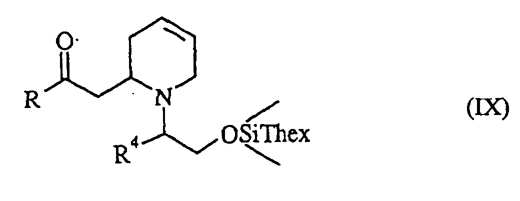 in which R, R⁴ and Thex are as defined above,
the totality of the compounds (VIII) and (IX) forming the compound of formula (X): 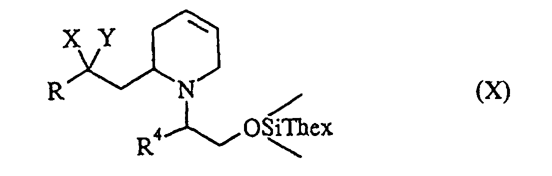 in which R, R⁴, X, Y and Thex are as defined for formula (I) and R⁴ is as defined above,
which compound of formula (X) is hydrolysed in an acid medium to yield a compound of formula (I/a), a particular case of the compounds of formula (I): 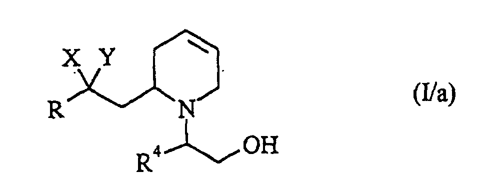 in which R, X, Y and R⁴ are as defined above,
which is:
- either treated, when X represents a hydrogen atom and Y represents a hydroxy group, with hydrogen in the presence of palladium-on-carbon to give a compound of formula (I/b), a particular case of the compounds of formula (I): 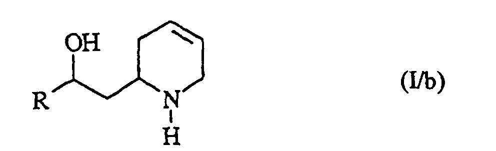 in which R is as defined above,
- or treated with a diphenylalkylsulfonium tetrafluoroborate of formula (XI): 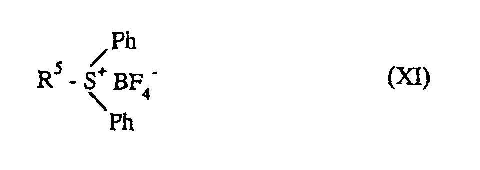 in which R⁵ represents a linear or branched (C₁-C₆)alkyl group,
to yield a compound of formula (XII): 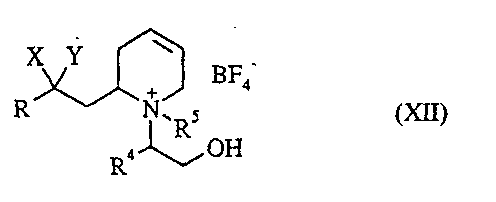 in which R, R⁴, R⁵, X and Y are as defined above,
which is subjected to the action of potassium tert-butanolate in tert-butanol to yield a compound of formula (I/c), a particular case of the compounds of formula (I): 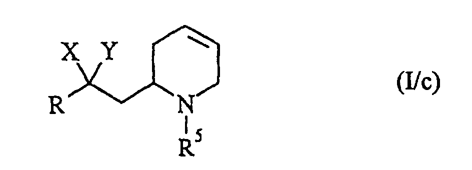 in which R, X, Y and R⁵ are as defined above,
which, when X represents a hydrogen atom and Y represents a hydroxy group, is treated with metachloroperbenzoic acid to give a compound of formula (XIII): 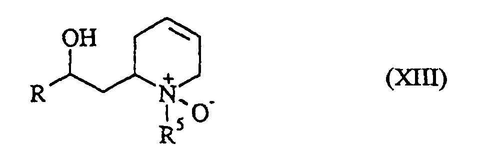 in which R and R⁵ are as defined above,
which is subjected to the action of trifluoroacetic anhydride to yield a compound of formula (XIV): 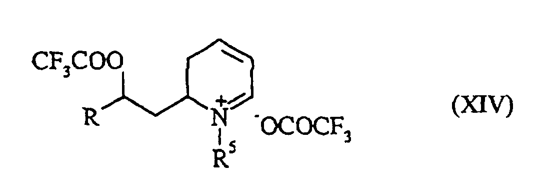 in which R and R⁵ are as defined above,
which is treated with BrZnCH₂COOR⁶, wherein R⁶ represents a linear or branched (C₁-C₆)alkyl group, to give a compound of formula (I/d), a particular case of the compounds of formula (I): 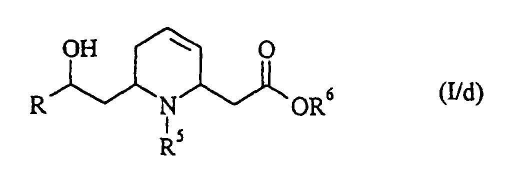 in which R, R⁵ and R⁶ are as defined above,
which is subjected in succession to Weinreb's reagent 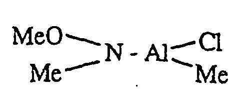 and then to the action of a lithium compound R⁷-Li, wherein R⁷ represents a phenyl group optionally substituted by one or more halogen atoms or one or more linear or branched (C₁-C₆)alkyl groups, (C₁-C₆)alkoxy groups, linear or branched (C₁-C₆)polyhaloalkyl groups, hydroxy groups, to yield a compound of formula (I/e), a particular case of the compounds of formula (I): 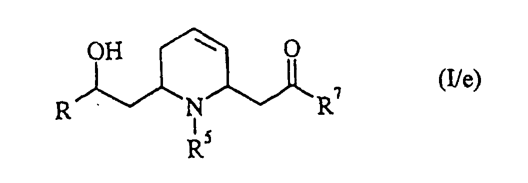 in which R, R⁵ and R⁷ are as defined above,
which is:
- either subjected to an oxidising agent to yield a compound of formula (I/f), a particular case of the compounds of formula (I): 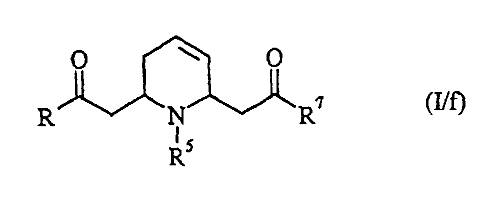 in which R, R⁵ and R⁷ are as defined above,
- or subjected to a reducing agent to yield a compound of formula (I/g), a particular case of the compounds of formula (I): 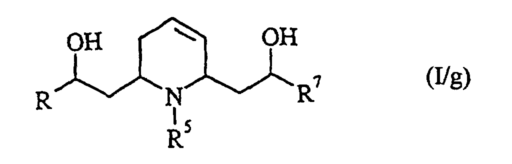 in which R, R⁵ and R⁷ are as defined above,
- or protected in acetal form by the action of an alcohol in an acid medium, to give a compound of formula (I/h), a particular case of the compounds of formula (I): 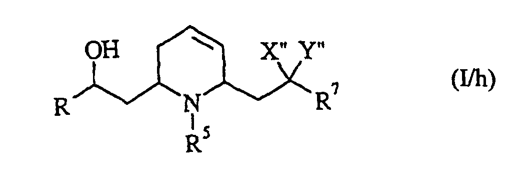 in which R, R⁵ and R⁷ are as defined above and X" and Y" simultaneously represent a linear or branched (C₁-C₆)alkoxy group or together form a linear or branched (C₁-C₄)-alkylenedioxy group,
the compounds (I/a) to (I/h) forming the totality of the compounds of the invention, which are purified, where appropriate, by a conventional purification technique, and which may, if desired, be separated into their various optical isomers or converted into a salt with a pharmaceutically acceptable acid or base.

7. Compounds of formulae (V), (XII) and (XIII) according to claim 6, for use as intermediates in the synthesis of compounds of formula (I) according to claim 1.

8. Pharmaceutical compositions comprising at least one compound of formula (I) according to claims 1 to 5, or an addition salt thereof with a pharmaceutically acceptable acid, in combination with one or more inert, non-toxic, pharmaceutically acceptable excipients or carriers.

9. Pharmaceutical compositions according to claim 8 comprising at least one active ingredient according to any one of claims 1 to 5, for use in the treatment of memory defects associated with cerebral ageing and with neurodegenerative diseases such as Alzheimer's disease, Parkinson's disease, Pick's disease, Korsakoffs disease, and frontal and subcortical dementia.
